# EUROPÄISCHE PATENTSCHRIFT

(11) **EP 1 450 760 B1**
(45) Veröffentlichungstag und Bekanntmachung des Hinweises auf die Patenterteilung: **14.03.2007**
(21) Anmeldenummer: 02792781.3
(22) Anmeldetag: 21.11.2002
(51) Int. Cl.: A61K 8/34, A61K 8/39, A61K 8/86, A61Q 17/00, A61Q 19/00, A61P 17/04, A61K 31/08

(54) **JUCKREIZSTILLENDE KOSMETISCHE UND DERMATOLOGISCHE ZUBEREITUNGEN**
ITCH-RELIEVING COSMETIC AND DERMATOLOGICAL PREPARATIONS
PREPARATIONS COSMETIQUES ET DERMATOLOGIQUES POUR APAISER LES DEMANGEAISONS

(30) Priorität: 27.11.2001 DE 10158199
(43) Veröffentlichungstag der Anmeldung: 01.09.2004
(73) Patentinhaber: Beiersdorf AG, 20245 Hamburg (DE)
(72) Erfinder: FILBRY, Alexander, 22459 Hamburg (DE); KRÖPKE, Rainer, 22869 Schenefeld (DE); ZELLE, Dagmar, 21640 Bliedersdorf (DE)
(86) Internationale Anmeldenummer: PCT/EP2002/013033
(87) Internationale Veröffentlichungsnummer: WO 2003/045349

(56) Entgegenhaltungen:
- WO-A-01/87274
- WO-A-93/25209
- WO-A-97/07821
- WO-A-02/102327
- DE-A- 19 824 681
- US-A- 4 767 617
- US-A- 5 853 740

## Beschreibung

Die vorliegende Erfindung betrifft juckreizstillende kosmetische und dermatologische Zubereitungen, die Polidocanol und ätherisches Öl enthält. Es wird bei Anwendung der erfindungsgemäßen Zubereitung eine sofortige und langanhaltende Juckreizminderung festgestellt.

Juckreiz (Hautjucken, Pruritus) ist eine subjektiv lästige und unangenehme, auf die Haut oder Schleimhaut bezogene Sinneswahrnehmung. Sie kann örtlich begrenzt oder aber auch auf den ganzen Körper ausgedehnt vorkommen. Juckreiz kann brennenden, stechenden oder kribbelnden Charakter haben und führt fast immer zu Reiben oder Kratzen. Dadurch können wiederum Hauterscheinungen wie Kratzspuren, offene Wunden, Krusten, Hautinfektionen entstehen. Der Juckreiz wird über Schmerzrezeptoren der Haut wahrgenommen und über das vegetative Nervensystem zum Gehirn geleitet. Die Ursache des Juckreizes ist oft vielfältig und kann manchmal als erstes und einziges Symptom bei Hauterkrankungen oder Allgemeinerkrankungen auftreten. Neben trockener Haut, fehlender Feuchtigkeitszufuhr, Kleidungseinflüssen kann Juckreiz aber auch durch äußere Einwirkungen und Hautreizungen, wie z.B. durch Stiche von Mücken oder nach Kontakt mit Brennnesseln oder Qualen entstehen. Juckreiz kann eine Reaktion auf chemische, mechanische oder thermische Reize sein. Es können als Folge der äußerlichen Reizung, wie z.B. durch Einwirkungen chemischer Substanzen, z.B. Histamin (Mückenstich), Apamin (Bienenstich), durch allergische Immunreaktion, durch Druck oder Reibung oder auch durch Wärme oder Sonnenbestrahlung, Quaddeln, Urtikaria und andere mit Juckreiz verbundene Hautreaktionen hervorgerufen werden. Bekannt und wesentlich ist auch der sogenannte senile Pruritus, der bei älteren Menschen oft zu unangenehmen Begleiterscheinung führt.

Bei der Behandlung des Juckreizes stehen neben diagnostischen Ursachenfindung vor allem juckreizmindernde bzw. -stillende Maßnahmen im Vordergrund. Dieses geschieht oft unabhängig des Einflusses auf die zugrundeliegende Erkrankung.

Die bekannten Medikamente oder Linderungsmittel gegen Juckreiz werden in Tablettenform oder in Form von Puder, Salben, Gelen oder Emulsionen verabreicht. Während Tabletten zu einer systemischen Wirkung führen, die eine Belastung des gesamten Organismus nach sich zieht, ist bei Gelen, Cremes und Emulsionen eine spezifische Formulierung notwendig um den Wirkstoff dauerhaft und beständig und vor allem wirksam in der Zubereitung vorzuhalten.

Gegen Juckreiz gibt es zahlreiche erprobte Mittel. So werden überwiegend kühlende Gele und Stifte eingesetzt, die neben der Kühlung durch verdunstenden Alkohol oder Wasser auch Antihistamine enthalten. Ferner wird Isoprenalin verwendet, was jedoch aufgrund der bekannten Hautreizung nicht zu empfehlen ist.

Eine bekannte Creme ist beispielweise Camillen 60 FUDES. Die Creme auf Basis von Vaseline, Calendulaöl und Menthol zur Linderung von Juckreiz. Zusätze wie die antibakteriellen Substanzen, Benzoesäure und Triclosan schützen vorbeugend vor Pilzbefall.

Das von der Firma Desitin vertriebene schmerzstillende Gel Thesit® enthält die Bestandteile Polidocanol, Mepivacain-hydrochlorid und Benzalkoniumchlorid. Das Gel ist für die Behandlung von Hautverletzungen, Verbrennungen und Juckreiz vorgesehen, wobei Mepivacain-hydrochlorid als Lokalanästhetikum wirkt.

Aus DE 19833177 ist die Anwendung eines Pflaster mit den Wirkstoffen Menthol und Benzocain, als Lokalanästhetikum, zur Reizminderung bei Insektenstichen und Brennnesselkontakt beschrieben. Nachteilig ist, dass Lokalanästhetika in der Lage sind Kontaktallergien auszulösen und der Zusatz von Lokalanästhetika den späteren Vertrieb als Kosmetikum unzulässig macht.

WO 9325209 offenbart eine O/W-Emulsion mit Polidocanol, Thymianöl und Bisabolol sowie Polyoxyethylenfettsäureester als O/W-Emulgatoren, die eine juckreizstillende Wirkung aufweisen soll.

US 4767617 offenbart eine Dauerwellenemulsion mit Melissenöl, Polidocanol und Polyoxysorbitanmonolaurat als O/W-Emulgator.

US 5853740 offenbart ein Drug Delivery System auf Basis einer Mikroemulsion umfassend Lecithin, Laureth-9 und Pfefferminzöl.

Aufgabe der vorliegenden Erfindung ist es eine Zubereitung zur Verfügung zu stellen, die langanhaltend juckreizmindemd bzw.- juckreizstillend ist, den Stand der Technik bereichert und eine Alternative zu den bislang bekannten juckreizmindernden Zubereitungen bietet. Weitere Aufgabe ist es eine juckreizstillende Zubereitung zur Verfügung zu stellen, die die Nachteile der aus dem Stand der Technik Zubereitungen nicht aufweist und insbesondere eine sofortige und langanhaltende juckreizstillende Wirkung aufweist, ohne das eine übermäßige Menge an juckreizstillenden Wirkstoffen zugegeben werden muß. Weitere Aufgabe ist es eine juckreizstillende Zubereitung zur Verfügung zu stellen, die pflegend wirkt und anwendungsfreundlich appliziert werden kann. Sie muss die Befeuchtung und Feuchtigheitsregulierung der Haut normalisieren und auch vor Hautschäden durch Sonnenbestrahlung schützen. Insbesondere besteht in Länder hoher Sonnenexposition zumeist auch die Gefahr vor Insektenstichen und/oder Quallenberührungen.

Gelöst werden die angeführten Aufgaben durch Zubereitungen gemäß dem Hauptanspruch. Gegenstand der Unteransprüche sind vorteilhafte Ausführungsformen der erfindungsgemäßen Zubereitungen. Des weiteren umfasst die Erfindung die Verwendung derartiger Zubereitungen.

Es war überraschend und für den Fachmann nicht vorauszusehen, dass eine Zubereitung enthaltend ein oder mehrere O/W-Emulgatoren, gegebenenfalls ein oder mehrere Coemulgatoren, Polyethylenglycol (9) laurylether (Polidocanol) und ätherisches Öl, die gestellten Aufgaben löst, wobei als O/W-Emulgator Polyethylenglycol (12) cetearylether und/oder Monostearate Polyoxyethylene (40) gewählt werden.

Insbesondere erweisen sich die Wirkstoffe Polidocanol und Menthol in nichtionischen Emulsionsgrundlagen, insbesondere in Emulsionen auf Basis von Eumulgin B-1, überraschenderweise äußerst wirkungsvoll.

Die erfindungsgemäßen Zubereitungen führen zu einer deutlich wirksamen, langanhaltenden und auch subjektiv merklichen Reduzierung des Juckreizes auf der Haut.

Polidocanol ist eine Kurzbezeichnung für Polyethylenglykol(9)monododecylether, ein Addukt von 9 Mol Ethylenoxid an den Dodecylalkohol; es entspricht der Formel 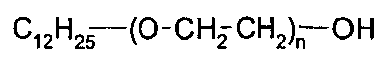 wobei n einen Durchschnittswert von 9 annimmt und das durchschnittliche Molgewicht etwa 600 g/mol beträgt. Polidocanol ist auch bekannt unter Nonaoxythylene Monododecyl Ether, PEG-9, Lauryl Ether, Polyethylene Glycol 450, Lauryl Ether Polyoxyethylene (9). Die Handelsbezeichnungen und Vertriebsfirmen sind u.a. Alfonic 1412-9.0 Ethoxylate (Condea Vista), Atlas G-4829 (Uniqema Americas), Calgene Nonionic L-9 (Calgene), Carsonon L-9 (Lonza Inc./Lonza Ltd.), Carsonon L-985 (Lonza Inc./Lonza Ltd.), Hetoxol L-9 (Heterene), Hetoxol LS-9 (Heterene), Jeecol LA-9 (Jeen), Marlipal 24/90 (Condea Chemie (Marl)), Nikkol BL-9EX (Nikko), Procol LA-9 (Protameen), Sympatens-AU090 (Kolb), Unicol LA-9 (Universal Preserv-A-Chem).

In Gegenwart von Elektrolyten wird die Emulgierbarkeit des Polidocanols nicht beeinflusst. Polidocanol ist eine amphiphile Verbindung mit hoher Kapillaraktivität. Wässrige Lösungen zeigen eine hohe Oberflächenaktivität und dementsprechend ein gutes Spreitvermögen auf der Haut. In wässrigen Lösungen angewendet, zeigt Polidocanol starke lokalanaesthetische Eigenschaften (K. Söhring u. Mitarb., Arch. Int. pharmacodyn. 87, 301 [1951]; K. J. Siems u. K. Soehring, Arzneim.-Forschung./Drug Res. 2, 109 [1952]; K. Soehring u. Mitarb., Arch. Int. pharmacodyn. 91, 112 [1952]; H. S. Zipf u. Mitarb., Arzneim.-Forsch./Drug. Res. 7, 162 [1957]).

Polidocanol wird zu einem Anteil von 0,01 bis 20 Gew.%, insbesondere von 1 bis 10 Gew.%, in der Zubereitung eingesetzt.

Unter ätherischen Ölen sind aus Pflanzen gewonnene Konzentrate bekannt, die als natürliche Rohstoffe hauptsächlich in der Parfüm- und Lebensmittelindustrie eingesetzt werden und die mehr oder weniger aus flüchtigen Verbindungen bestehen, wie zum Beispiel echte ätherische Öle, Citrusöle, Absolues, Resinoide.
Oft wird der Begriff auch für die noch in den Pflanzen enthaltenen flüchtigen Inhaltsstoffe verwendet. Im eigentlichen Sinn versteht man aber unter ätherischen Ölen Gemische aus flüchtigen Komponenten, die durch Wasserdampfdestillation aus pflanzlichen Rohstoffen hergestellt werden.

Echte ätherische Öle bestehen ausschließlich aus flüchtigen Komponenten, deren Siedepunkt überwiegend zwischen 150 und 300 °C liegen. Anders als zum Beispiel fette Öle hinterlassen sie deshalb beim Auftupfen auf Filterpapier keinen bleibenden durchsichtigen Fettfleck. Ätherische Öle enthalten überwiegend Kohlenwasserstoffe oder monofunktionelle Verbindungen wie Aldehyde, Alkohole, Ester, Ether und Ketone.

Stammverbindungen sind Mono- und Sesquiterpene, Phenylpropan-Derivate und längerkettige aliphatische Verbindungen.
Bei manchen ätherischen Öle dominiert ein Inhaltsstoff (zum Beispiel Eugenol in Nelkenöl mit mehr als 85%), andere sind wieder äußerst komplex zusammengesetzt. Oft werden die organoleptische Eigenschaften nicht von den Hauptkomponenten, sondern von Neben- oder Spurenbestandteilen geprägt, wie zum Beispiel von den 1,3,5-Undecatrienen und Pyrazinen im Galbanum-Öl. Bei vielen der kommerziell bedeutenden ätherischen Öle geht die Zahl der identifizierten Komponenten in die Hunderte. Sehr viele Inhaltsstoffe sind chiral, wobei sehr oft ein Enantiomer überwiegt oder ausschließlich vorhanden ist, wie zum Beispiel (-)-Menthol im Pfefferminzöl oder (-)-Linalylacetat im Lavendelöl.

Als besonders bevorzugtes ätherisches Öl hat sich das Menthol erwiesen. Menthol ist Bestandteil des bekannten japanischen Pfefferminzöls (CAS: 20747-49-3). Wichtigstes Isomer ist (-)-Menthol. Menthol erzeugt beim Einreiben auf die Haut infolge Oberflächenanästhesierung und Reizung der kälteempfindlichen Nerven bei Migräne oder dergleichen ein angenehmes Kältegefühl. Erwiesenermaßen zeigen die betroffenen Hautpartien aber normale bzw. erhöhte Temperatur.

Als weitere bevorzugte ätherische Öle können Oleum Eucalypti, Oleum Menthae piperitae, Oleum camphoratum, Oleum Rosmarini, Oleum Thymi, Oleum Pini sibricum und Oleum Pini silverstris sowie die Terpene 1,8-Cineol und Levomethanol sowie Oleum Abietis albae, Oleum Anisi, Oleum Aurantii Floris, Oleum Bergamottae, Oleum Calendulae infusum, Oleum Caryophylli, Oleum Chamomillae, Oleum Cinnamomi ceylanici, Oleum Citri, Oleum Citronellae, Oleum Cupressi, Oleum Cymbopogonis, Oleum Jecoris, Oleum Lavendulae, Oleum Macidis, Oleum Majoranae, Oleum Melaleucae viridiflorae, Oleum Melissae, Oleum Menthae arvensis, Oleum Millefolium, Oleum Myrrhae, Oleum Myrte, Oleum Pini sibricum, Oleum Pinisilvestris, Oleum Salviae, Oleum Santali, Oleum Terebinthinae rectificat., Oleum Valerianae und Oleum Zingiberis genannt werden.

Die ätherischen Öle werden einzeln oder in Kombination mit anderen zu einem Anteil von insgesamt 0,001 bis 10 Gew.%, insbesondere zu 0,01 bis 1 Gew.% in der Zubereitung eingesetzt.

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca. 1 µm bis ca. 50 µm. Solche "Makroemulsionen" sind, ohne weitere färbende Zusätze, milchigweißgefärbt und opak. Feinere "Makroemulsionen", deren Tröpfchendurchmesser im Bereich von ca. 10⁻¹ µm bis ca. 1 µm liegen, sind, wiederum ohne färbende Zusätze, bläulichweiß gefärbt und undurchsichtig.

Mizellaren und molekularen Lösungen mit Partikeldurchmessern kleiner als ca. 10⁻² µm, ist vorbehalten, klar und transparent zu erscheinen.

Der Tröpfchendurchmesser von transparenten bzw. transluzenten Mikroemulsionen dagegen liegt im Bereich von etwa 10⁻² µm bis etwa 10⁻¹ µm. Solche Mikroemulsionen sind meist niedrigviskos. Die Viskosität vieler Mikroemulsionen vom O/W-Typ ist vergleichbar mit der des Wassers.

Vorteil von Mikroemulsionen ist, dass in der dispersen Phase Wirkstoffe feiner dispers vorliegen können als in der dispersen Phase von "Makroemulsionen". Ein weiterer Vorteil ist, dass sie aufgrund ihrer niedrigen Viskosität versprühbar sind.
Nachteilig an den Mikroemulsionen des Standes der Technik ist, dass stets ein hoher Gehalt an einem oder mehreren Emulgatoren eingesetzt werden muss, da die geringe Tröpfchengröße eine hohe Grenzfläche zwischen den Phasen bedingt, welche in der Regel durch Emulgatoren stabilisiert werden muss.

Zu den erfindungsgemäß bevorzugten O/W - Emulgatoren zählen Eumulgin B-1 der Firma Cognis, eine Emulsionsgrundlage entsprechend den Bezeichnungen Polyethylenglycol(12)cetearylether, PEG-12 Cetyl/Stearyl Ether, Polyethylene Glycol 600 Cetyl/Stearyl Ether, Polyoxyethylene (12) Cetyl/Stearyl Ether. Diese Verbindung ist auch bekannt unter dem Namen CETEARETH-12 und wird unter folgenden Bezeichnungen von folgenden Firmen vertrieben: Atlas G-4822 (Uniqema Americas), Jeecol CS-12 (Jeen), Procol CS-12 (Protameen), Sabowax CS 11 (Sabo), Sympatens-ACS/120 (Kolb), Unimul-B-1 (Universal Preserv-A-Chem), Volpo CS12 (Croda Oleochemicals).
Ebenso vorteilhaft ist Myrj 52S von der Fa. Uniqema, ein Emulgator entsprechend den Bezeichnungen Macrogol Stearate 2000, Polyethylene Glycol 2000 Monostearate, Polyoxyethylene (40) Monostearate, Polyoxyl 40 Stearate, Stearethate 40. Diese Verbindung ist auch bekannt unter dem Namen PEG-40 STEARATE und wird unter folgenden Bezeichnungen von folgenden Firmen vertrieben: AEC PEG-40 Stearate (A & E Connock) Calgene POE (40) MS (Calgene) Crodet S40 (Croda Oleochemicals) Emerest 2715 (Henkel) Emerest 2715 (Henkel/COSPHA) Hetoxamate SA-40 (Heterene) Jeemate 2000-DPS (Jeen) Lanoxide-52 (Lanaetex) LIPOPEG-39-S (Lipo) Myrj 52 (Uniqema Americas) Myrj 52S (Uniqema Americas) Nikkol MYS-40 (Nikko) Pegosperse 1750 MS (Lonza Inc./Lonza Ltd.) Protamate 1540 DPS (Protameen) Protamate 2000 DPS (Protameen) Ritox 52 (RITA) ROL 52 (Fabriquimica) Sabowax SE 40 (Sabo) Simulsol M 52 (SEPPIC) Sipoic MS-40 (Specialty Industrial) Sympatens-BS/400 (Kolb) Tego Acid S 40 P (Goldschmidt) Unipeg-S-40 (Universal Preserv-A-Chem).

Als Coemulgator hat sich sehr vorteilhaft das Cutina GMS (Fa. Cognis) erwiesen, ein Coemulgator entsprechend der Bezeichnung 2,3-Dihydroxypropyl Octadecanoate, Glycerin 1-Stearate, Glyceryl Monostearate, Gycerol 1-Stearate, Jeechem HMS Monostearin, Octadecanoic Acid, 2,3-Dihydroxypropyl Ester Octadecanoic Acid, Monoester with 1,2,3-Propanetriol Stearic Acid 1-Monoglyceride. Diese Verbindung ist auch bekannt unter den Namen GLYCERYL STEARATE und wird auch unter den Bezeichnungen AEC Glyceryl Stearate (A & E Connock) Aldo HMS (Lonza Inc./Lonza Ltd.) Aldo MS (Lonza Inc./Lonza Ltd.) Aldo MSLG (Lonza Inc./Lonza Ltd.) Alkamuls GMS (Rhodia Inc.) Arlacel 129 (Uniqema Americas) Arlacel 161 (Uniqema Americas) Arlacel 169 (Uniqema Americas) Calgene GMS (Calgene) Capmul GMS (Abitec) Ceral Mex (Fabriquimica) Ceral MN (Fabriquimica) Ceral MNT (Fabriquimica) Cerasynt GMS (ISP Van Dyk) Cerasynt SD (ISP Van Dyk) Cithrol GMS N/E (Croda Oleochemicals) CPH-53-N (Hall) CPH-144-N (Hall) Cremophor GS 11 (BASF) Cutina GMS (Cognis) Emerest 2400 (Henkel) Emerest 2400 (Henkel/) ESTOL 1474 (Uniqema (Netherlands)) ESTOL 3740 (Uniqema (Netherlands)) Geleol (Gattefosse s.a.) Imwitor 191 (Condea Chemie (Witten)) Imwitor 900 (Condea Chemie (Witten)) Jeechem GMS-450 (Jeen) Jeechem HMS (Jeen) Kemester 5500 (Witco) Kemester 6000 (Witco) Kessco GMS (Akzo Nobel Surface Chemistry) KESSCO GMS (Stepan) KESSCO GMS 63F (Stepan) KESSCO GMS PURE (Stepan) Lanesta 24 (Lanaetex) vertrieben.

Weitere O/W-Emulgatoren bzw. Coemulgatoren sind polypropoxylierten O/W-Emulgatoren
- wobei der polyethooxylierte bzw. polypropoxylierte O/W-Co- und/oder Emulgatoren gewählt wird oder werden aus der Gruppe
- der Fettalkoholethoxylate der allgemeinen Formel R-O-(-CH₂-CH₂-O-)ₙ-H, wobei R einen verzweigten oder unverzweigten Alkyl-, Aryl- oder Alkenylrest und n eine Zahl von 10 bis 50 darstellen
- der ethoxylierten Wollwachsalkohole,
- der Polyethylenglycolether der allgemeinen Formel R-O-(-CH₂CH₂-O-)ₙ-R', wobei R und R' unabhängig voneinander verzweigte oder unverzweigte Alkyl- oder Alkenylreste und n eine Zahl von 10 bis 80 darstellen
- der Fettsäureethoxylate der allgemeinen Formel R-COO-(-CH₂CH₂-O-)ₙ -H, wobei R einen verzweigten oder unverzweigten Alkyl- oder Alkenylrest und n eine Zahl von 10 bis 40 darstellen,
- der veretherten Fettsäureethoxylate der allgemeinen Formel R-COO-(-CH₂-CH₂-O-)ₙ -R', wobei R und R' unabhängig voneinander verzweigte oder unverzweigte Alkyl- oder Alkenylreste und n eine Zahl von 10 bis 80 darstellen,
- der veresterten Fettsäureethoxylate der allgemeinen Formel R-COO-(-CH₂CH₂-O-)ₙ -C(O)-R', wobei R und R' unabhängig voneinander verzweigte oder unverzweigte Alkyl- oder Alkenylreste und n eine Zahl von 10 bis 80 darstellen,
- der Polyethylenglycolglycerinfettsäureester gesättigter und/oder ungesättigter, verzweigter und/oder unverzweiger Fettsäuren und einem Ethoxylierungsgrad zwischen 3 und 50,
- der ethoxylierten Sorbitanester mit einem Ethoxylierungsgrad von 3 bis 100
- der Cholesterinethoxylate mit einem Ethoxylierungsgrad zwischen 3 und 50,
- der ethoxylierten Triglyceride mit einem Ethoxylierungsgrad zwischen 3 und 150,
- der Alkylethercarbonsäuren der allgemeinen Formel R-O-(-CH₂-CH₂-O-)ₙ-CH₂-COOH bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 5 bis 30 darstellen,
- der Polyoxyethylensorbitolfettsäureester, basierend auf verzweigten oder unverzweigten Alkan- oder Alkensäuren und einen Ethoxylierungsgrad von 5 bis 100 aufweisend, beispielsweise vom Sorbeth-Typ,
- der Alkylethersulfate bzw. die diesen Sulfaten zugrundeliegenden Säuren der allgemeinen Formel R-O-(-CH₂-CH₂-O-)ₙ-SO₃-H mit kosmetisch oder pharmazeutisch akzeptablen Kationen, wobei R einen verzweigten oder unverzweigten Alkyl- oder Alkenylrest mit 5-30 C-Atomen und n eine Zahl von 1 bis 50 darstellen.
- der Fettalkoholpropoxylate der allgemeinen Formel R-O-(-CH₂-CH(CH₃)-O-)ₙ-H, wobei R einen verzweigten oder unverzweigten Alkyl- oder Alkenylrest und n eine Zahl von 10 bis 80 darstellen,
- der Polypropylenglycolether der allgemeinen Formel R-O-(-CH₂-CH(CH₃)-O-)ₙ-R', wobei R und R' unabhängig voneinander verzweigte oder unverzweigte Alkyl- oder Alkenylreste und n eine Zahl von 10 bis 80 darstellen
- der propoxylierten Wollwachsalkohole,
- der veretherten Fettsäurepropoxylate der allgemeinen Formel R-COO-(-CH₂-CH(CH₃)-O-)ₙ-R', wobei R und R' unabhängig voneinander verzweigte oder unverzweigte Alkyl- oder Alkenylreste und n eine Zahl von 10 bis 80 darstellen,
- der veresterten Fettsäurepropoxylate der allgemeinen Formel R-COO-(-CH₂-CH(CH₃)-O-)ₙ-C(O)-R', wobei R und R' unabhängig voneinander verzweigte oder unverzweigte Alkyl- oder Alkenylreste und n eine Zahl von 10 bis 80 darstellen,
- der Fettsäurepropoxylate der allgemeinen Formel R-COO-(-CH₂-CH(CH₃)-O-)ₙ-H, wobei R einen verzweigten oder unverzweigten Alkyl- oder Alkenylrest und n eine Zahl von 10 bis 80 darstellen,
- der Polypropylenglycolglycerinfettsäureester gesättigter und/oder ungesättigter, verzweigter und/oder unverzweiger Fettsäuren und einem Propoxylierungsgrad zwischen 3 und 80
- der propoxylierten Sorbitanester mit einem Propoxylierungsgrad von 3 bis 100
- der Cholesterinpropoxylate mit einem Propoxylierungsgrad von 3 bis 100
- der propoxylierten Triglyceride mit einem Propoxylierungsgrad von 3 bis 100
- der Alkylethercarbonsäuren der allgemeinen Formel R-O-(-CH₂-CH(CH₃)O-)ₙ-CH₂-COOH bzw. deren kosmetisch oder pharmazeutisch akzeptablen Salze, wobei R einen verzweigten oder unverzweigten Alkyl- oder Alkenylrest und n eine Zahl von 3 bis 50 darstellen,
- der Alkylethersulfate bzw. die diesen Sulfaten zugrundeliegenden Säuren der allgemeinen Formel R-O-(-CH₂-CH(CH₃)-O-)ₙSO₃-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen,
- der Fettalkoholethoxylate/propoxylate der allgemeinen Formel R-O-Xₙ-Yₘ-H, wobei R einen verzweigten oder unverzweigten Alkyl- oder Alkenylrest darstellen, wobei X und Y nicht identisch sind und jeweils entweder eine Oxyethylengruppe oder eine Oxypropylengruppe und n und m unabhängig voneinander Zahlen von 5 bis 50 darstellen,
- der Polypropylenglycolether der allgemeinen Formel R-O-Xₙ-Yₘ-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der veretherten Fettsäurepropoxylate der allgemeinen Formel R-COO-Xₙ-Yₘ-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der Fettsäureethoxylate/propoxylate der allgemeinen Formel R-COO-Xₙ-Yₘ-H, wobei R einen verzweigten oder unverzweigten Alkyl- oder Alkenylrest, wobei X und Y nicht identisch sind und jeweils entweder eine Oxyethylengruppe oder eine Oxypropylengruppe und n und m unabhängig voneinander Zahlen von 5 bis 50 darstellen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)-stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20), Polyethylenglycol(13)cetylether (Ceteth-13), Polyethylenglycol(14)cetylether (Ceteth-14), Polyethylengfycol(15)cetylether (Ceteth-15-), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20), Polyethylenglycol-(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol-(18)isocetylether (Isoceteth-18), Polyethylenglycol(19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12-), Polyethylenglycol(12)isolaurylether (Isolaureth-12), Polyethylenglycol(13)cetylstearyl-ether (Ceteareth-13), Polyethylenglycol(14)cetylstearylether (Ceteareth-14), Polyethylenglycol(1 5)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20).

Es ist ferner von Vorteil, die Fettsäureethoxylate aus folgender Gruppe zu wählen: Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(12)isostearat, Polyethylenglycol(13)isostearat, Polyethylenglycol-(14)isostearat, Polyethylenglycol(15)isostearat, Polyethylenglycol(16)isostearat, Polyethylenglycol(17)isostearat, Polyethylenglycol(18)isostearat, Polyethylenglycol(19)iso-stearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat.

Als ethoxylierte Alkylethercarbonsäure bzw. deren Salz kann vorteilhaft das Natriumlaureth-11-carboxylat verwendet werden.
Als Alkylethersulfat kann Natrium Laureth 1-4 sulfat vorteilhaft verwendet werden.
Als ethoxyliertes Cholesterinderivat kann vorteilhaft Polyethylenglycol(30)Cholesteryl-ether verwendet werden. Auch Polyethylenglycol(25)Sojasterol hat sich bewährt.
Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)-sorbitanmonooleat zu wählen.

Bei den erfindungsgemäßen Zubereitungen zeigt sich eine unerwartete synergistische Wirkstoffentfaltung durch die Kombination der Wirkstoffe Polidocanol und ätherischen Ölen, insbesondere Menthol, mit den ausgewählten O/W-Emulgatoren. Die ausgewählten O/W-Emulgatoren bauen ein Gelnetzwerk in der kohärenten wässrigen Phase in der Form aus, welche die Freisetzung der Wirkstoffe Polidocanol und ätherisches Öl derart begünstigt, das sich die juckreizstillende Wirkung sofort und dennoch nachhaltig entfalten kann.

Die klinische Prüfung der Wirksamkeit der erfindungsgemäßen Zubereitung fällt ausgesprochen positiv aus und bestätigt die vorteilhaften Eigenschaften der Zubereitungen. Im Rahmen einer klinisch kontrollierten Studie wurden erfindungsgemäße Zubereitungen mit 1 Gew.% Menthol und 2 Gew.% Polidocanol, 0,5 Gew.% Menthol und 2 Gew.% Polidocanol und ein Placebo an mehreren Personen im Alter zwischen 18 und 60 Jahren getestet.
Das Resultat:
- Sofortige Juckreizminderung nach Auftragung der Zubereitung
- Intensivierung der Juckreizminderung innerhalb von 30 Minuten nach Auftragung bis zum fast vollständigen Wegfall des Reizes
- Nach einer Woche Behandlung auch sichtbare Reduzierung der Juckreize
- Kein Unterschied zwischen den eingesetzten erfindungsgemäßen Zubereitungen

Weitere Zusätze der erfindungsgemäßen Zubereitungen sind neben Wasser übliche in der Kosmetik einsetzbare Stoffe wie nachfolgend näher beschrieben.

Als Ölphase der erfindungsgemäßen Zubereitung können Öle mit unterschiedlicher . Polarität, Molekulargewicht und Struktur eingesetzt werden. Die Ölkomponente oder die Gesamtheit der Ölkomponenten der erfindungsgemäßen Zubereitung wird bevorzugt gewählt aus der Gruppe der Ester aus gesättigten und/oder ungesättigten, verzweigten und/oder unverzweigten Alkancarbonsäuren oder Hydroxyalkancarbonsäuren einer Kettenlänge von 1 bis 44 C-Atomen und gesättigten und/oder ungesättigten, verzweigten und/oder unverzweigten Alkoholen einer Kettenlänge von 1 bis 44 C-Atomen, aus der Gruppe der Ester aus aromatischen Carbonsäuren und gesättigten und/oder ungesättigten, verzweigten und/oder unverzweigten Alkoholen einer Kettenlänge von 1 bis 30 C-Atomen sofern die Ölkomponente oder die Gesamtheit der Ölkomponenten bei Raumtemperatur eine Flüssigkeit darstellen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die Ölphase kann vorteilhaft gewählt werden aus der Gruppe der verzweigten und unverzweigten Kohlenwasserstoffe, der Silikonöle, Lanoline, der Adipinsäureester, der Butylenglycoldiester, der Dialkylether oder -carbonate, der Gruppe der gesättigten oder ungesättigten, verzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.
Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Sehr vorteilhaft sind beispielsweise Dicapylylcarbonat, Dicaprylylether, Paraffinöl, Coco Caprylat/Caprat, Caprylic/Capric Triglycerid, Cyclomethicon, Dimethicon, Octyldodecanol sowie natürliche Öle wie z.B. Sojaöl, Macadamiaöl, Nachtkerzenöl. Ferner können auch sehr vorteilhaft Ölmischungen eingesetzt werden wie beispielsweise Paraffinöl/Dicaprylyether, Paraffinöl/CocoCaprylat-Caprat.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Halistar AB*) und/oder Diethylhexylnaphthalat (*Hallbrite TQ*).

Die Ölkomponenten können vorteilhaft in einem Gehalt von 1 bis 50 Gew.%, bezogen auf die Gesamtzubereitung vorliegen, bevorzugt sind etwa 1 bis 40 Gew.%.

Durch die Verwendung von Wachsen (Esterwachse, Triglyceridwachse, ethoxlierte Wachse usw.) in der Ölphase oder als Ölphase lässt sich die Zubereitung nochmals verbessern. Bevorzugt ist es, wenn die Wachskomponente oder die Gesamtheit der Wachskomponenten gewählt wird aus der Gruppe der Ester aus gesättigten und/oder ungesättigten, verzweigten und/oder unverzweigten Alkancarbonsäuren oder Hydroxycarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, aus der Gruppe der Ester aus aromatischen Carbonsäuren und gesättigten und/oder ungesättigten, verzweigten und/oder unverzweigten Alkoholen einer Kettenlänge von 1 bis 80 C-Atomen sofern die Wachskomponente oder die Gesamtheit der Wachskomponeriten bei Raumtemperatur einen Festkörper darstellen, der natürlichen Wachse, der Diester von Polyolen und C10-C80 Fettsäuren, der ethoxylierten Wachse, der Triglyceridwachse, der C16-C60 Fettsäuren und/oder C16-C80 Fettalkohole, der Mineralölwachse.
Insbesondere vorteilhaft können die Wachskomponenten aus der Gruppe der C₁₆₋₃₆-Alkylstearate, der C₁₀₋₄₀-Alkylstearate, der C₂₀₋₄₀Alkylisostearate, der C₂₀₋₄₀-Dialkyldimerate, der C₁₈₋₃₈-Alkylhydroxystearoylstearate, der C20-40 Dialkyldimerate, der C₂₀₋₄₀-Alkylerucate gewählt werden, ferner C₃₀₋₅₀-Alkylbienenwachs, Cetylpalmitat, Methylpalmitat, Cetearylbehenat, Octacosanyl Stearate. Auch Siliconwachse wie beispielsweise Stearyltrimethylsilan/Stearylalkohol sind gegebenenfalls vorteilhaft.

Vorteilhaft im Sinne der vorliegenden Erfindung sind außerdem Esterwachse, die Ester aus
1. gesättigten und/oder ungesättigten, verzweigten und/oder unverzweigten Mono- und/oder Dicarbonsäure mit 10 bis 50 Kohlenstoffatomen, bevorzugt 15-45 Kohlenstoffatomen und
2. Glycerin
   darstellen. Dabei können Mono-, Di- und Triglyceride vorteilhaft sein.

**Besonders vorteilhaft sind die im folgenden aufgelisteten Glyceride:**

| **Glycerid** | **Handelsname** | **erhältlich bei** |
|---|---|---|
| C₁₆₋₁₈ -Triglycerid | Cremeol HF-52-SPC | Aarhus Oliefabrik |
| Glycerylhydroxystearat | Naturchem GMHS | Rahn |
| Hydrierte Coco-Glyceride | Softisan 100 | Hüls AG |
| Caprylisäure/Caprinsäure/Isostearinsäure/ Adipinsäure Triglycerid | Softisan 649 | Dynamit Nobel |
| C₁₈₋₃₈ Triglycerid | Syncrowax HGLC | Croda GmbH |
| Glyceryltribehenat | Syncrowax HRC | Croda GmbH |
| Glyceryl-tri-(12-hydroxystearat) | Thixcin R | Rheox / NRC |
| Hydriertes Ricinusöl | Cutina HR | Cognis AG |
| C₁₆₋₂₄ -Triglycerid | Cremeol HF-62-SPC | Aarhus Oliefabrik |

Besonders bevorzugt ist, die Wachskomponenten zu wählen aus der Gruppe der Triglyceridwachse wie C18-38 Triglycerid oder Tribehenin zu wählen. Ferner hat sich herausgestellt, dass ethoxylierte Wachse wie beispielsweise PEG-8 Bienenwachs, PEG 6 Sorbitanbienenwachs, PEG-2 hydrogeniertes Castoroil, PEG-12 Carnaubawachs vorteilhaft sind.

Außerdem können die erfindungsgemäßen Zubereitungen Farbstoffe und/oder Pigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Pigmente können organischen und anorganischen Ursprungs sein, wie beispielsweise organische vom Azo-Typ, Indigoide, Triphenylmethan-artige, Anthrachinone, und Xanthin Farbstoffe, die als D&C and FD&C blues, browns, greens, oranges, reds, yellows bekannt sind. Anorganische Pigmente bestehen aus unlöslichen Salzen von zertifizierten Farbstoffen, die als Lakes oder Eisenoxide bezeichnet werden. Beispielsweise können Barium lakes, calcium lakes, aluminum lakes, titandioxide, mica and iron oxides Verwendung finden. Als Al-Salze sind z.B Red 3 Aluminum Lake, Red 21 Aluminum Lake, Red 27 Aluminum Lake, Red 28 Aluminum Lake, Red 33 Aluminum Lake, Yellow 5 Aluminum Lake, Yellow 6 Aluminum Lake, Yellow 10 Aluminum. Lake, Orange 5 Aluminum Lake, Blue 1 Aluminum Lake und Kombinationen einsetzbar.
Als Eisenoxide oder -oxidhydrate sind z.B. cosmetic yellow oxide C22-8073 (Sunchemical) cosmetic oxide MC 33-120 (Sunchemical), cosmetic brown oxide C33-115 (Nordmann& Rassmann), cosmetic russet oxide C33-8075 (Sunchemical) bekannt und gegebenfalls vorteilhaft. Als Alumosilicat ist ultramarinblau (Les colorants Wacker) einsetzbar.

Auch Perlglanzpigmente lassen sich in die erfindungsgemäßen Emulsionen einarbeiten. Diese sind beipsielsweise von den Firmen Costenoble (Cloisonne-Typ, Flamenco-Typ, Low Lustre-Typ), Merck (Colorona-Typen, Microna-Typ, Timiron-Typ, Colorona, Ronasphere), Les Colornats Wacker (Covapure, Vert oxyde de Chrome), Cadre (Colorona, Sicopearl), BASF (Sicopearl, Sicovit), Rona (Colorona) bekannt. Als besonders vorteilhaftes Perlglanzpigment haben sich beispielsweise Timiron Silk Gold und Colorona Red Gold bewährt.

Vorteilhafte Farbpigmente sind weiterhin Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. (Die Stoffe sind nach ihrer Colour Index Number geordnet.)

| Colour Index Number | Deutsche Bezeichnung | CAS-Nr. bzw. Summenformel |
|---|---|---|
| 10316 | C-ext. Gelb 1 | 846-70-8 |
| 12075 | C-Orange | 3468-63-1 |
| 14700 | C-Rot 57 | 4548-53-2 |
| 15510 | C-ext.Orange 8 | 633-96-5 |
| 15585 | C-Rot 55 | 2092-56-0 |
| 15585:1 | C-Rot 55 | 5160-02-1 |
| 15800:1 | C-ext. Rot 57 | 6371-76-2 |
| 15850 | Lithol Rubin 8 | 5858-81-1 |
| 15850:1 | C-Rot 12 | 5281-04-9 |
| 15880:1 | C-ext. Rot 61 | 6417-83-0 |
| 15980 | C-Orange 9 | C₁₆H₁₀N₂O₇S₂· 2Na |
| 15985 | C-Orange 10 | 2783-94-0 |
| 16035 | C-Rot 60 | 29956-17-6 |
| 17200 | C-Rot 58 | C₁₆H₁₃N₃O₇S₂ · 2Na |
| 19140 | C-Gelb 10 | 1934-21-0 |
| 20170 | C-ext. Braun 4 | 1320-07-6 |
| | | 6371-84-2 |
| 26100 | C-ext. Rot 56 | 85-86-9 |
| 42053 | C-Grün 12 | C₃₇H₃₆N₂O₁₀S₃ · 2Na |
| 42090 | C-Blau 21 | 2650-18-2 |
| 42090 | C-Blau 21 | 2650-18-2 |
| | (Ammonium Salz) | 6371-85-3 |
| | | 37307-56-5 |
| 45170 | C-Rot 59 | 81-88-9 |
| 45170:1 | (Rhodamin B-stearat) | C₂₈H₃₁N₂O₃ · C₁₈H₃₅O₂ |
| 45370:1 | C-Rot 27 | C₂₀H₁₀Br₂O₅ |
| 45380 | C-Rot 30 | 17372-87-1 |
| 45380:2 | Tetrabromfluoreszein | 15086-94-9 |
| 45410 | C-Rot 34 | 18472-87-2 |
| 45410:1 | Tetrabromtetrachlorfluoreszein | 13473-26-2 |
| 45425 | C-Rot 35 | C₂₀H₁₀l₂O₅ · 2Na |
| 45425:1 | Fluoreszein-Gemisch | 518-40-7 |
| | | 38577-97-8 |
| 47000 | C-ext. Gelb 23 | 8003-22-3 |
| 47005 | C-Gelb 11 | 8004-92-0 |
| 59040 | C-ext. Gelb 24 | 6358-69-6 |
| 60725 | C-ext. Violett 18 | 81-48-1 |
| 61565 | C-Grün 10 | 128-80-3 |
| 61570 | C-Grün 11 | 4403-90-1 |
| 73360 | C-Rot 28 | 2379-74-0 |
| 75120 | C-Orange 12 | 8015-67-6 |
| 75130 | C-Orange 11 | 7235-40-7 |
| 75170 | Guanin | 68-94-0 |
| | | 73-40-50 |
| 75470 | C-Rot 50 | C₂₂H₂₀O₁₃ |
| 75480 | Henna | C₁₀H₆O₃ (Lawson) |
| 75810 | C-Grün 8 | 11006-34-1 |
| 75810 | C-Grün 7 | 479-61-8 |
| | | 519-62-0 |
| 77000 | C-Pigment 1 | Al |
| 77007 | C-Blau 16 | 57455-37-5 |
| 77019 | C-Weiß 11 | 12001-26-2 |
| 77288 | C-Grün 9 | 1308-38-9 |
| 77289 | C-Grün 14 | 12001-99-9 |
| 77400 | Bronze | 7440-50-8 |
| 77491 | C-Rot 45 | 1309-37-1 |
| 77492 | C-Braun 3 | Fe₂O₃ FeO(OH) |
| | (C-Gelb 8) | |
| 77499 | C-Schwarz 5 | Fe₃O₄ |
| 77510/20 | C-Blau 17 | C₆FeN₆ · 4/3 Fe |
| 77742 | C-Violett 11 | 10101-66-3 |
| 77820 | C-Pigment 2 | 7440-22-4 |
| 77891 | C-Weiß 7 | 13463-67-7 (TiO₂) |
| 77947 | C-Weiß 8 | 1314-13-2 |

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden.

Die Liste der genannten Farbstoffe und Farbpigmente, die in den erfindungsgemäßen Emulsionen verwendet werden können, soll selbstverständlich nicht limitierend sein.

Erfindungsgemäße Zubereitungen können auch Puderstoffe enthalten. Als Puderstoffe werden beispielsweise Wismuthoxichlorid, titanisierter Glimmer, Siliciumdioxid (fumed silica), spherische Siliciumdioxid-Perlen, Polymethylmethacyrlat-Perlen, micronisiertes Teflon, Bornitrid, Acrylatpolymere, Aluminumsilicat, Aluminum-Stärke-Octenylsuccinat, Bentonit, Calciumsilicat, Cellulose, Kreide, Maisstärke, Glycerylstärke, Hectorit, hydrisiertes Silica, Kaolin, Magnesiumhydroxide, Magnesiumoxid, Magnesiumsilicate, Magnesiumtrisilicat, Maltodextrin, Montmorillonit, microcristalline Cellulose, Reisstärke, Silica, Talk, Mica, Titaniumdioxid, Zinklaurate, Zinkmyristat, Zinkneodecanoat, Zinkrosinat, Zinkstearat, Polyehtylen, Aluminiumoxid, Attapulgit, Calciumcarbonat, Calciumsilicat, Dextran, Kaolin, Nylon, Silicasilylat, Seidenpuder, Serecit, Zinnoxid, Titaniumhydroxid, Trimagnesiumphosphat, Wallnußschalenpuder oder beliebige Mischungen eingesetzt werden.

Die genannten üblichen kosmetischen Zusätze erlauben es, ohne das die Wirksamkeit der Zubereitung sinkt, über einen längeren Zeitraum ohne äußerlich sichtbare Einbußen auf der Haut getragen zu werden.

Weiterhin lassen sich weitere vorteilhafte Wirkstoffe in die Zubereitungen einarbeiten. So erweist sich beispielsweise Spitzwegerichtinktur als erneut wirksamkeitssteigemd bezüglich der Juckreizminderung gegenüber den Zubereitungen ohne Spitzwegerichtinktur.

Zusätzlich können Pflegewirkstoffe eingearbeitet werden, welche sich nicht auf die fettlöslichen Wirkstoffe beschränken, sondern auch aus der Gruppe der wasserlöslichen Wirkstoffe gewählt werden können, beispielsweise Vitamine und dergleichen mehr. Somit ergibt sich ein pflegender Einfluss auch bei längerer Tragedauer.

Eine erstaunliche Eigenschaft der erfindungsgemäße Zubereitungen ist, dass diese sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können.

Die Zubereitungen enthalten daher vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.
Besonders vorteilhaft werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Pentat-, Hexa-, Heptahioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol deren Derivate, Vitamin C und Derivate (z.B. Ascorbylpalmitate, Mg - Ascorbylphosphate, Ascorbylacetate), Isoascorbinsäure und ihre Derivate, Tocopherole und Derivate (z.B. Vitamin E - acetat), Vitamin A und Derivate (Vitamin A - palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Sofern Vitamin E und/oder dessen Derivate das oder die Antioxidantien darstellen, ist es vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.%, bezogen auf das Gesamtgewicht der Formulierung, zu wählen.

Erfindungsgemäß können Wirkstoffe auch sehr vorteilhaft gewählt werden aus der Gruppe der lipophilen Wirkstoffe, insbesondere aus folgender Gruppe:
Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linolensäure, Ölsäure, Eicosapentaensäure, Docosahexaensäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl®, Eucerit® und Neocerit®.

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen. Bevorzugter NO-Synthasehemmer ist das Nitroarginin.

Weiter vorteilhaft werden der oder die Wirkstoffe gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspostitionen angegeben):

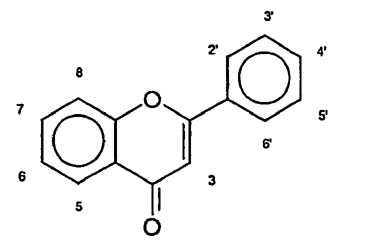

Einige der wichtigeren Flavone, welche auch bevorzugt in erfindungsgemäßen Zubereitungen eingesetzt werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt aus der Gruppe der Substanzen der generischen Strukturformel 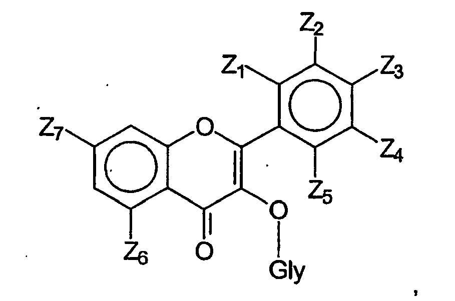 wobei Z₁ bis Z₇ unabhängig voneinander gewählt werden aus der Gruppe H, OH, Alkoxy- sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 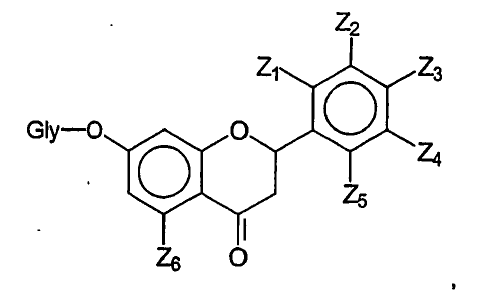 wobei Z₁ bis Z₆ unabhängig voneinander gewählt werden aus der Gruppe H, OH, Alkoxy- sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 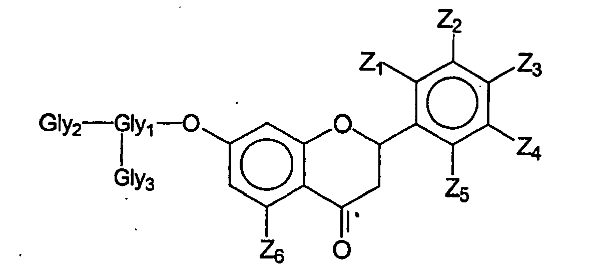 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur

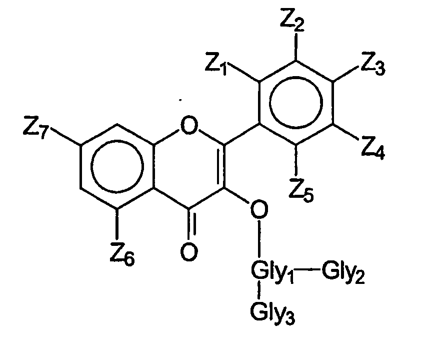

Besonders vorteilhaft werden die erfindungsgemäßen Flavonglycoside aus der Gruppe, welche durch die folgende Struktur wiedergegeben werden: 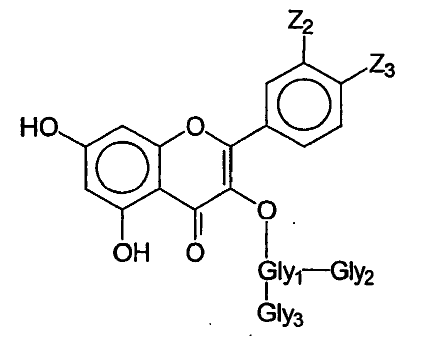 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin. Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflavanon-7-glucosid) und Isoquercetin (3,3',4',5,7-Pentahydroxyflavanon-3-(β-D-Glucopyranosid).

Vorteilhaft ist es auch, den oder die Wirkstoffe aus der Gruppe der Ubichinone und Plastochinone zu wählen.

Ubichinone zeichnen sich durch die Strukturformel 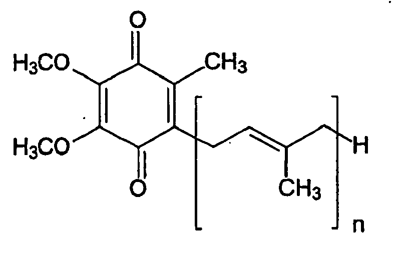 aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

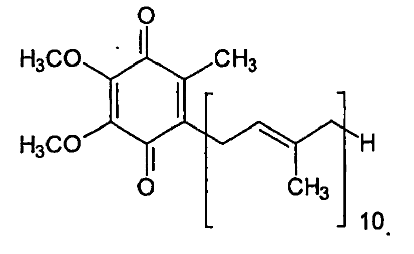

Plastochinone weisen die allgemeine Strukturformel 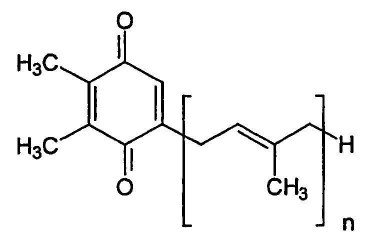 auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate sind bevorzugte Wirkstoffe im Sinne der vorliegenden Erfindung. Kreatin zeichnet sich durch folgende Struktur aus: 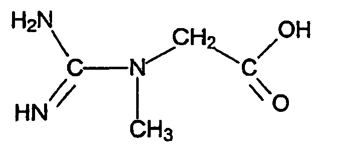 Bevorzugte Derivate sind Kreatinphosphat sowie Kreatinsulfat, Kreatinacetat, Kreatin-ascorbat und die an der Carboxylgruppe mit mono- oder polyfunktionalen Alkoholen veresterten Derivate.

Ein weiterer vorteilhafter Wirkstoff ist L-Camitin [3-Hydroxy-4-(trimethylammonio)-butter-säurebetain]. Auch Acyl-Carnitine, welche gewählt aus der Gruppe der Substanzen der folgenden allgemeinen Strukturformel 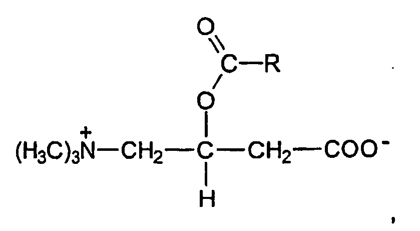 wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcamitin und insbesondere Acetylcamitin. Beide Entantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.
Weitere vorteilhafte Wirkstoffe sind Sericosid, Pyridoxol, Aminoguadin, Phytochelatin, Isoflavone (Genistein, Daidzein, Daidzin, Glycitin), Niacin, Tyrosinsulfat, Dioic Acid, Adenosin, Pyridoxin, Arginin, Vitamin K, Biotin und Aromastoffe, Sericosid wie auch Wirkstoffkombinationen der genannten Wirkstoffe.

Darüber hinaus ist eine der wichtigsten Aufgaben kosmetischer und/oder dermatologischer Zubereitungen die Befeuchtung und Feuchtigheitsregulierung der Haut. Zu diesem Zwecke werden den Zubereitungen neben Wasser als Bestandteil aller Emulsionen sogenannte Feuchthaltemittel (engl. Moisturizer) zugefügt. Synthetische Feuchthaltemittel sind Ersatzstoffe für den natürlichen Feuchthaltefaktor (engl. Natural Moisturizing Factor NMF), der aus 40 % freien Aminosäuren, 12 % Pyroglutaminsäure, 12 % Lactaten, 7 % Harnstoff, 1,5 % Hamsäure sowie Glucosamin, Kreatinin und verschiedenen Salzen besteht.
Als synthetische Feuchthaltemittel werden neben hydrolysierten Proteinen vor allem Polyole (mehrwertige Alkohole) verwendet.

Der wichtigste Vertreter der Polyole ist das Glycerin (Glycerol, 1,2,3-Propantriol), eine farb- und geruchlose, süß schmeckende Flüssigkeit. Glycerin besitzt die folgende Struktur:

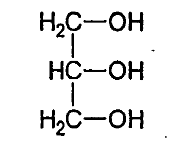

Ein weiterer wichtiger Vertreter der Polyole ist das Sorbit, ein fünfwertiger Alkohol, der in Vogelbeeren vorkommt und synthetisch durch Reduktion von Glucose gewonnen werden kann. Sorbit ist durch folgende Struktur gekennzeichnet:

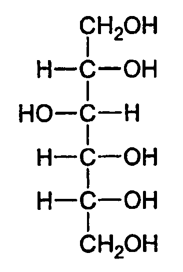

Insbesondere können auch Moisturizer Chitosan, Fucogel, Milchsäure, Propylenglycol, Sorbitol, Polyethylenglycol, Dipropylenglycol, Butylenglycol, Mannitol, Natriumpyrolidoncarbonsäure, Glycin Hyaluronsäure und deren Salze, Aminosäuren wie Glycin, Harnstoff, Natrium und Kaliumsalze leichter an die Haut gebunden werden. Durch die Verwendung von Wachsen (Esterwachse, Triglyceridwachse, ethoxlierte Wachse usw.) in der Ölphase oder als Ölphase lässt sich die Anhaftung der Wirkstoffe und Moisturizer nochmals verbessern.

Der Feuchtigkeitsgehalt der Haut kann mittels corneometrischer Messungen bestimmt werden. Hierbei werden mit Hilfe eines Corneometers die dielektrischen Eigenschaften des Stratum corneums untersucht. Das Corneometer besteht aus einem Streukondensator, dessen Kapazität durch die dielektrischen Eigenschaften des Stratum corneums (mit-) bestimmt wird. Um zu ermitteln, wie lang die durch eine kosmetische und/oder dermatologische Zubereitung bewirkte Hautbefeuchtung anhält, wird bei konstanten Messbedingungen der Feuchtigkeitsgehalt der Haut jeweils vor Anwendung sowie zwei Stunden nach Anwendung der kosmetischen und/oder dermatologischen Zubereitung bestimmt. Es hat sich bei diesen Untersuchungen herausgestellt, dass die Moisturizer enthaltende erfindungsgemäße Zubereitung den Feuchtigkeitshaushalt der Haut positiv beeinflusst, was äußerst angenehm für die durch Hautreizung geschädigte Haut.

Die Liste der genannten zusätzlichen Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Es ist gegebenenfalls möglich und vorteilhaft, die erfindungsgemäßen Zubereitungen als Grundlage für pharmazeutische Formulierungen zu verwenden. Mutatis mutandis gelten entsprechende Anforderungen an die Formulierung medizinischer Zubereitungen. Die Übergänge zwischen reinen Kosmetika und reinen Pharmaka sind dabei fließend. Als pharmazeutische Wirkstoffe sind erfindungsgemäß grundsätzlich alle Wirkstoffklassen geeignet, wobei lipophile Wirkstoffe bevorzugt sind. Beispiele sind: Antihistaminika, Antiphlogistika, Antibiotika, Antimykotika, die Durchblutung fördernde Wirkstoffe, Keratolytika, Antihistaminika, Antiphlogistika, Antibiotika, Antimykotika, die Durchblutung fördernde Wirkstoffe, Keratolytika, Hormone, Steroide, Vitamine, Hormone, Steroide, Vitamine usw.
Es ist gegebenenfalls möglich und vorteilhaft, in die erfindungsgemäßen Zubereitungen Repellentien einzuarbeiten. Besonders vorteilhafte Repellent-Wirkstoffe im Sinne der vorliegenden Erfindung sind die obengenannten Wirkstoffe N,N-Diethyl-3-methylbenzamid, 3-(N-n-Butyl-N-acetyl-amino)propionsäureethylester, 1-Piperidincarbonsäure-2-(2-hydroxyethyl)-1-methylpropylester und Dimethylphthalat.

Es ist auch vorteilhaft im Sinne der vorliegenden Erfindung, kosmetische und dermatologische Zubereitungen zu erstellen, deren Zweck auch der Schutz vor Sonnenlicht ist. So werden gewöhnlich UV-A- bzw, UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar.
Dementsprechend enthalten die Zubereitungen im Sinne der vorliegenden Erfindung vorzugsweise mindestens eine UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Besonders vorteilhafte bei Raumtemperatur flüssige UV-Filtersubstanzen im Sinne der vorliegenden Erfindung sind Homomenthylsalicylat (INCl: Homosalicylate), 2-Ethylhexyl-2-cyano-3,3-diphenylacrylat (INCl: Octocrylene), 2-Ethylhexyl-2-hydroxybenzoat (2-Ethylhexylsalicylat, Ethylhexylsalicylat, INCl: Ethylhexyl Salicylate) und Ester der Zimtsäure, vorzugsweise 4-Methoxyzimtsäure(2-ethylhexyl)ester (2-Ethylhexyl-4-methoxycinnamat, INCl: Ethylhexyl Methoxycinnamate) und 4-Methoxyzimtsäureisopentylester (Isopentyl-4-methoxycinnamat, INCl: Isoamyl p-Methoxycinnamate), 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| ZnO NDM | 5% Dimethicone | H&R |
| MZ- 303S | 3% Methicone | Tayca Corporation |
| MZ- 505S | 5% Methicone | Tayca Corporation |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid /Stearinsäure | Tayca Corporation |
| Eusolex T-2000 | Alumina / Simethicone | Merck KgaA |
| Titandioxid T805 (Uvinul TiO₂) | Octyltrimethylsilan . | Degussa |
| MT-100AQ | Silica / Aluminiumhydroxid / Alginsäure | Tayca Corporation |
| Eusolex T-Aqua | Wasser/ Alumina / Natriummetaphosphat | Merck KgaA |

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCl-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCl Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendi-methylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornyliden-methyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCl: Bis-Ethylhexyloxyphenol Methoxyphenyl Triazine), welches unter der Handelsbezeichnung Tinosorb® S bei der CIBA-Chemikalien GmbH erhältlich ist;
- Diethylhexylbutylamidotriazon (INCl: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCl: Ethylhexyl Triazone), welches von der BASF Aktiengesellschaft unter der Warenbezeichnung UVINUL® T 150 vertrieben wird.

Weitere im Sinne der vorliegenden Erfindung vorteilhafte Triazinderivate sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyl-oxy)-2-hydroxy]-phenyl}-6-(4-methoxyphenyl)-1,3,5-triazin und das 2,4-Bis-{[4-(1',1',1' ,3',5',5',5'-Heptamethylsiloxy-2"-methyl-propyloxy)-2-hydroxy]-phenyl}-6-(4-methoxyphenyl)-1,3,5-triazin.

Ein vorteilhaftes Benzotriazol im Sinne der vorliegenden Erfindung ist das 2,2'-Methylen-bis-(6-(2H-benzotriazol-2-yl)-4-(1,1,3,3-tetramethylbutyl)-phenol), ein Breitbandfilter, welcher durch die chemische Strukturformel 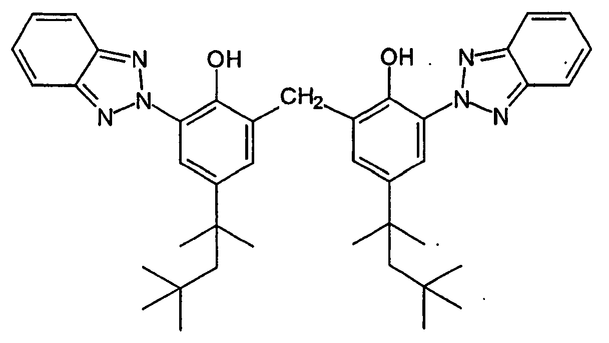 gekennzeichnet ist und unter der Handelsbezeichnung Tinosorb® M bei der CIBA-Chemikalien GmbH erhältlich ist.

Ein vorteilhaftes Benzotriazol im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]-propyl]-phenol (CAS-Nr.: 155633-54-8) mit der INCl-Bezeichnung Drometrizole Trisiloxane, welches durch die chemische Strukturformel 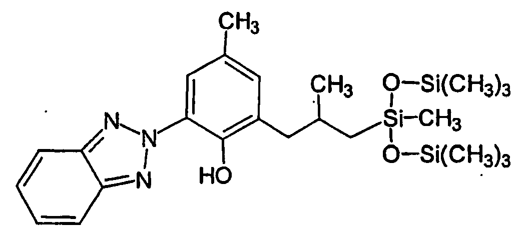 gekennzeichnet ist.

Weitere vorteilhafte Benzotriazole im Sinne der vorliegenden Erfindung sind [2,4'-Dihydroxy-3-(2H-benzotriazol-2-yl)-5-(1,1,3,3-tetramethylbutyl)-2'-octoxy-5'-benzol]diphenylmethan, 2,2' Methylen-bis-[6-(2H-benzotriazol-2-yl)-4-(methyl)phenol], 2,2'-Methylene-bis-[6-(2H-benzotiazol-2-yl)-4-(1,1,3,3-trtramethylbutyl)phenol], 2-(2'-Hydroxy-5'-octylphenyl)-benzotriazol, 2-(2'-Hydroxy-3',5'-di-t-amylphenyl)benzotriazol und 2-(2'-Hydroxy-5'-methylphenyl)benzotriazol.

Die weiteren UV-Filtersubstanzen können öllöslich oder wasserlöslich sein.

Vorteilhafte öllösliche UV-B- und/oder Breitband-Filtersubstanzen im Sinne der. vorliegenden Erfindung sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist.

Auch zeigte sich das Hydroxybenzophenone, insbesondere Aminobenzophenone, in Kombination mit Acrylamid-Polymeren oder -Copolymeren geeignete Lichtschutzfilter darstellen und daher vorteilhafterweise in der erfindungsgemäßen Zubereitung enthalten sein können.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.% bis 30 Gew.%, vorzugsweise 0,5 bis 20 Gew.%, insbesondere 1,0 bis 15,0 Gew.%, jeweils bezogen auf das Gesamtgewicht der Zubereitungen, um kosmetische Zubereitungen zur Verfügung zu stellen, die die Haut vor dem gesamten Bereich der ultravioletten Strahlung schützen.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Bakterizide, Parfüme, Substanzen zum Verhindern des Schäumens, Verdickungsmittel und/oder Füllstoffe, die das Hautgefühl verbessern.

Die Applikationsform der erfindungsgemäßen Zubereitung kann wahlweise als Gel, Creme, Lotion, Spray, als Drug Delivery system oder über getränkte Tücher eingestellt werden.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrükkenbindungen zwischen Geliermittel und Dispersionsmittel, aber auch zwischen Dispersionsmittelmolekülen untereinander verwirklicht sind, können zu starker Vemetzung auch des Dispersionsmittels führen. Hydrogele können zu fast 100 % aus Wasser bestehen (neben beispielsweise ca. 0,2- 1,0 % eines Geliermittels) und dabei durchaus feste Konsistenz besitzen. Der Wasseranteil liegt dabei in eisähnlichen Strukturelementen vor, so dass Gele daher ihrer Namensherkunft [aus lat. "gelatum" = "Gefrorenes" über den alchimistischen Ausdruck "gelatina" (16. Jhdt.) für nhdt. "Gelatine"] durchaus gerecht werden.

In der kosmetischen und pharmazeutischen Galenik sind ferner auch Lipogele und Oleogele (aus Wachsen, Fetten und fetten Ölen) sowie Carbogele (aus Paraffin oder Petrolatum) geläufig. In der Praxis unterscheidet man Oleogele, welche praktisch wasserfrei vorliegen, Hydrogele, welche praktisch fettfrei sind. Meistens sind Gele durchsichtig. In der kosmetischen bzw. pharmazeutischen Galenik zeichnen sich Gele in aller Regel durch halbfeste, oft fließfähige Konsistenz aus.

Ferner sind sogenannte Tensidgele gebräuchliche Zubereitungen des Standes der Technik. Darunter versteht man Systeme, die neben Wasser eine hohe Konzentration an Emulgatoren aufweisen, typischerweise mehr als ca. 25 Gew.-%, bezogen auf die Gesamtzusammensetzung. Solubilisiert man in diese Tensidgele, fachsprachlich auch "surfactant gels" genannt, Ölkomponenten, werden Mikroemulsionsgele erhalten, welche auch als "ringing gels" bezeichnet werden. Durch Zusatz von nichtionischen Emulgatoren, beispielsweise Alkylpolyglycosiden, lassen sich kosmetisch elegantere Mikroemulsionsgele erhalten.

Es ist an sich bekannt, die Tröpfchen einer niedrigviskosen, insbesondere dünnflüssigen Mikroemulsion mit vernetzenden Substanzen miteinander zu verknüpfen, um auf diese Weise das dreidimensionale Netzwerk eines Geles zu erhalten.

Unter Creme versteht man eine nicht freifließende Zubereitung, die eine Viskosität, bei 25°C, von mehr als 10.000 mPa*s aufweist (Viskotester VT-02, Fa. Haake). Unter Lotion versteht man eine freifließende Zubereitung, die eine Viskosität, bei 25°C, von 2000-10.000 mPa*s aufweist (Viskotester VT-02, Fa. Haake)..

Ferner lassen sich die erfindungsgemäßen Zubereitungen sehr gut als Tränkungsmedium für Tücher und Gewebe nutzen, die nass oder trocken vom Verbraucher angewendet werden können. Die Kompositionen mit den ultrafeinen Tröpfchen ziehen dabei leicht auf die Faser auf, was als Vorteil anzusehen ist.

Auch ist es möglich die erfindungsgemäßen Zubereitungen in Form von Drug Delivery Systemen bereit zu stellen. Drug Delivery Systeme sind pflasterartige Arzneiformen, die den Wirkstoff in Form eines Reservoirs enthalten, aus dem die Wirkstofffreisetzung über einen längeren Zeitraum nach 0. Ordnung erfolgt. Aufgrund dieser Eigenschaften sind die Drug Delivery Systeme in der Lage, den Organismus unabhängig von der Wirkstoffkonzentration im Reservoir über einen langen Zeitraum mit konstanten Mengen Wirkstoff pro Zeiteinheit zu versorgen und werden daher der Gruppe von Retardarzneimitteln zugeordnet.

Drug Delivery Systeme werden zwischen Topical und Transdermal Delivery unterschieden. Die topicalen Formulierungen enthalten Wirkstoffe, deren Freisetzung und Wirkung auf den Bereich direkt unterhalb und der Umgebung des Orts der Applikation begrenzt ist. Die transdermalen Formulierungen hingegen enthalten Wirkstoffe, die durch die Haut appliziert werden, um durch das Eindringen in das Gefäßsystem im gesamten Organismus einen wirksamen Wirkstoffspiegel zu erzeugen. Die Drug Delivery Systeme haben gegenüber anderen Arzneiformen (z.B. Salbe, Sprays, Suppositorien, Tabletten) eine Reihe von Vorteilen:
- Aufgrund der langen Wirkungsdauer der Drug Delivery Systeme wird die Compliance der Patienten im Vergleich zu Arzneiformen, die mehrmals im Verlauf eines Tages appliziert werden müssen, deutlich verbessert.
- Durch die Applikation der Drug Delivery Systeme kann eine Senkung der Dosis erfolgen, wodurch eine Verminderung der Nebenwirkungen von Wirkstoffen, die z.B. eine geringe therapeutische Breite aufweisen, ermöglicht wird.
- Beim Auftreten von Nebenwirkungen kann die Applikation des Wirkstoffs durch die Entfernung des Drug Delivery Systems schlagartig gestoppt werden.
- Durch die konstante Freisetzung des Wirkstoffs werden die Schwankungen der Wirkstoffkonzentration, die bei einer wiederholten Applikation am Tag auftreten, im Bereich der Haut und im Serum verhindert.
- Die transdermale Applikation reduziert im Vergleich zu oralen Arzneiformen den firstpass Metabolismus des Arzneistoffs, da der Metabolismus im Bereich der Haut im Vergleich zum Metabolismus im Magen und Leber deutlich geringer ist. Dies hat zur Folge, dass die Dosis der mit Drug Delivery Systemen applizierten Arzneistoffe geringer ist im Vergleich zu den oralen Arzneiformen.
- Bei den Drug Delivery Systemen muss im Gegensatz zu den oralen Arzneimitteln nicht mit einem Einfluss der Nahrung auf die Penetration des Arzneistoffs gerechnet werden.
- Die Dosierung kann sehr leicht über die Fläche der Drug Delivery Systeme definiert werden.

Die Herstellung der erfindungsgemäßen Zubereitungen erfolgt vorteilhaft auf die Weise, daß ein Gemisch aus den Grundkomponenten, umfassend Wasserphase, Ölphase, einen oder mehrere der erfindungsgemäßen O/W-Emulgatoren, gewünschtenfalls einen oder mehrerer Co-Emulgatoren, sowie gewünschtenfalls weitere Hilfs-, Zusatz- und/oder Wirkstoffe, auf eine Temperatur bringt, welche mindestens der Schmelztemperatur der höchstschmelzenden Ölkomponente entspricht, und die gebildete Emulsion hernach auf Raumtemperatur abkühlt, wobei erfindungsgemäß der oder die Wirkstoffe Polidocanol und ätherische Öle und eventuell Verdicker zu jedem Zeitpunkte der Herstellung beigefügt werden können. Der gesamte Vorgang vollzieht sich bevorzugt unter Rühren.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

**O/W-Emulsionen**

| | 1 | 2 | 3 | 4 | 5* |
|---|---|---|---|---|---|
| Cetylalkohol | 1,0 | 0,5 | 1,0 | 2,0 | 3,0 |
| Glycerylstearat | 1,0 | 1,5 | 1,0 | 3,0 | 2,0 |
| Paraffinöl | 2,5 | 1,0 | 4,0 | 2,5 | 1,5 |
| Decyloleat | 0,5 | 0,75 | --- | 2,0 | 0,25 |
| Octyldodecanol | --- | 1,0 | --- | 3,0 | 0,25 |
| Dicaprylylcarbonat | 0,1 | 0,05 | --- | 0,5 | --- |
| Glycerin | 3,0 | 5,0 | 12,0 | 15,0 | 1,5 |
| Dimethicon | 0,6 | 0,3 | 1,0 | 1,2 | 1,8 |
| Parfum | q,s, | q,s, | q,s, | q,s, | q,s, |
| Nachtkerzenöl | 2,0 | --- | 1,0 | --- | 8,0 |
| Capryl-/Caprinsäuretriglycerid | 2,0 | 2,5 | 6,0 | 5,0 | 0,5 |
| Methylparaben | 0,1 | 0,15 | -- | 0,03 | 0,14 |
| Polydocanol | 1,0 | 1,0 | 2,0 | 2,5 | --- |
| Menthol | 0,05 | --- | 0,15 | 0,25 | 0,3 |
| Polyethylenglycol(12)cetearylether | 1,0 | 2,2 | 2,6 | 2,4 | --- |
| Cyclomethicone | 0,5 | 0,75 | 1,0 | 2,0 | 0,25 |
| Ethanol | 1 | 5 | --- | 3 | 2 |
| modifizierte Stärke | --- | 2,5 | --- | 0,15 | --- |
| Propylparaben | 0,1 | 0,15 | --- | 0,15 | --- |
| lodopropynylbutylcarbamat + DMDM Hydantoin | --- | -- | 0,3 | --- | 0,1 |
| Wasser | ad 100 | ad 100 | ad 100 | ad 100 | ad 100 |

**O/W-Emulsionen**

| | 6* | 7 | 8* | 9* | 10 |
|---|---|---|---|---|---|
| PEG-40-Stearat | --- | 0,5 | --- | --- | 1,0 |
| Panthenol | --- | 0,75 | 1,0 | 2,0 | 0,25 |
| Octyldodecanol | --- | 1,0 | 0,75 | 3,0 | 0,25 |
| Dicaprylylcarbonat | --- | 0,05 | 0,15 | 0,5 | 1,0 |
| Glycerin | 3,0 | 5,0 | 10,0 | 15,0 | 1,5 |
| Milchsäure | 0,6 | 0,3 | 0,15 | 1,2 | 1,8 |
| Parfum | q,s, | q,s, | q,s, | q,s, | q,s, |
| Polydocanol | 3,0 | 1,0 | 0,5 | 2,5 | 5,0 |
| Menthol | 0,1 | 1,0 | 0,5 | 0,25 | 0,5 |
| Cetylalkohol | 1,0 | 0,5 | 1,0 | 2,0 | 3,0 |
| Glycerylstearat | 1,0 | 1,5 | 1,0 | 3,0 | 2,0 |
| Kaliumsorbat | --- | --- | 0,3 | --- | 0,15 |
| Capryl-/Caprinsäuretriglycerid | --- | 2,5 | 3,0 | 5,0 | 0,5 |
| Methylparaben | --- | 0,15 | 0,05 | 0,3 | 0,4 |
| Propylparaben | --- | 0,4 | 0,25 | 0,15 | --- |
| Wasser | ad 100 | ad 100 | ad 100 | ad 100 | ad 100 |

**O/W-Emulsion**

| | 11* | 12* | 13* | 14* | 15* |
|---|---|---|---|---|---|
| Cetylalkohol | 1,0 | 0,5 | 1,0 | 2,0 | 3,0 |
| Glycerylstearat | 1,0 | 1,5 | 1,0 | 3,0 | 2,0 |
| Polydocanol | 1,0 | 1,0 | 1,5 | 0,5 | 0,75 |
| Menthol | 0,5 | 0,1 | 0,05 | 0,25 | 0,2 |
| Paraffinöl | 2,5 | --- | 8,0 | 2,5 | 1,5 |
| Cocoglycerid | 8,0 | 6,0 | 0,5 | --- | 2,5 |
| PEG-40 hydriertes Rizinusöl | 1,0 | 1,5 | --- | 1,8 | --- |
| hydriertes Polyisobuten | --- | 0,75 | 1,0 | 2,0 | 0,25 |
| Octyldodecanol | 0,5 | 1,0 | 0,75 | 8,0 | 0,25 |
| Dicaprylylcarbonat | 0,1 | 0,05 | 0,15 | 0,5 | 1,0 |
| Glycerin | 3,0 | 5,0 | --- | 15,0 | 10,0 |
| Parfum | q,s, | q,s, | q,s, | q,s, | q,s, |
| Butylenglykol | 5,0 | --- | 15,0 | --- | 8,0 |
| Capryl-/Caprinsäuretriglycerid | 2,0 | 2,5 | 3,0 | 5,0 | 0,5 |
| Methylparaben | 0,4 | --- | 0,05 | 0,3 | 0,4 |
| Propylparaben | 0,3 | 0,4 | 0,25 | 0,15 | --- |
| Wasser | ad 100 | ad 100 | ad 100 | ad 100 | ad 100 |

**O/W-Emulsionen**

| | 16 | 17 | 18* | 19* | 20* |
|---|---|---|---|---|---|
| Polyethylenglycol(12)cetearylether | 1,0 | 0,5 | --- | --- | --- |
| Cyclomethicone | 0,5 | 0,75 | 1,0 | 2,0 | 0,25 |
| Octyldodecanol | 0,5 | 1,0 | 0,75 | 3,0 | 0,25 |
| Dicaprylylcarbonat | 0,1 | 1,0 | 15,0 | 0,5 | 1,0 |
| Dimethicon | --- | --- | --- | 5,0 | 1,0 |
| Glycerin | 3,0 | 5,0 | 10,0 | 15,0 | 1,5 |
| Polydocanol | 1,0 | 1,0 | 5 | 2,5 | 10 |
| Lanolinalkohol | 0,25 | 0,5 | 1,5 | 3,0 | 1,0 |
| Menthol | 0,05 | 0,5 | 0,25 | 0,3 | 0,5 |
| Paraffinöl | 2,5 | 1,0 | 8,0 | 2,5 | 1,5 |
| Parfum | q,s, | q,s, | q,s, | q,s, | q,s, |
| Tocopherol | 1,0 | 0,5 | 1,5 | 3,0 | 0,5 |
| Capryl-/Caprinsäuretriglycerid | 2,0 | 2,5 | 3,0 | 5,0 | 0,5 |
| Methylparaben | 0,4 | 0,15 | 0,05 | 0,3 | 0,4 |
| Propylparaben | 0,3 | 0,4 | 0,25 | 0,15 | --- |
| modifizierte Stärke | --- | 2,5 | --- | 0,15 | 5,0 |
| Wasser | ad 100 | ad 100 | ad 100 | ad 100 | ad 100 |

| | | | | | |
|---|---|---|---|---|---|
| * Vergleichsbeispiele | | | | | |

Alle aufgeführten beispielhaften Zubereitungen zeigen nach Auftragen auf die gereizte Haut eine sofortige Juckreizminderung bis hin zum Juckreizverlust. Weiterhin sind die erfindungsgemäßen Zubereitungen leicht auf die Haut aufzubringen und lassen sich angenehm verteilen.

## Patentansprüche

1. Kosmetische Zubereitung enthaltend
- Wasser,
- ein oder mehrere O/W-Emulgatoren,
- gegebenenfalls ein oder mehrere Coemulgatoren,
- eine oder mehrere Ölphasen
- Polyethylenglycol(9)laurylether und
- ätherisches Öl,
**dadurch gekennzeichnet, dass** als O/W-Emulgator Polyethylenglycol(12)cetearylether und/oder Monostearate Polyoxyethylene (40) enthalten ist.

2. Kosmetische Zubereitung auf nichtionischer Emulsionsgrundlage enthaltend Polyethylenglycol(12)cetearylether, Polyethylenglycol(9)laurylether und Menthol.

3. Kosmetische Zubereitung nach Anspruch 1 mit Monostearate Polyoxyethylene(40) als O/W-Emulgator.

4. Zubereitung nach Anspruch 1 oder 3 mit Glyceryl Stearate als Coemulgator

5. Zubereitung nach Anspruch 1, **dadurch gekennzeichnet, dass** die O/W-Emulgatoren bzw. Coemulgatoren ausgewählt sind aus der Gruppe der ethoxylierten Fettalkohole, ethoxylierten Stearate und/oder Glycerinester.

6. Zubereitung nach einem der vorstehenden Ansprüche mit Menthol als ätherisches Öl.

7. Zubereitung nach einem der vorstehenden Ansprüche, **dadurch gekennzeichnet, dass** Polyethylenglycol(9)laurylether zu einem Anteil von 0,01 bis 20 Gew,%, insbesondere von 1 bis 10 Gew.%, und/oder die ätherischen Öle einzeln oder in Kombination zu einem Anteil von 0,001 bis 10 Gew.%, insbesondere zu 0,01 bis 1 Gew.%, in der Zubereitung eingesetzt sind.

8. Zubereitung nach einem der vorstehenden Ansprüche mit einem Gehalt an Ölphase von 1 bis 50 Gew.%, insbesondere 1 bis 40 Gew.%, bezogen auf die Masse der Gesamtzubereitung.

9. Zubereitung nach einem der vorstehenden Ansprüche, **dadurch gekennzeichnet, dass** zusätzlich Spitzwegerichtinktur enthaltend ist,

10. Zubereitungen nach Anspruch 1 bis 9, **dadurch gekennzeichnet, dass** als Moisturizer Polyole wie insbesondere Glycerin, Sorbit, Harnstoff und/oder Butylenglykol zugesetzt sind.

11. Zubereitung nach einem der vorstehenden Ansprüche, **dadurch gekennzeichnet, dass** übliche kosmetische oder dermatologische Hilfsstoffe, wie Farbstoffe, Pigmente, Puderstoffe, hydrophile und/oder lipophile Wirkstoffe, Desodorantien, Lichtschutzmittel und/oder sonstige Hilfsstoffe zugesetzt sind.

12. Zubereitung nach Anspruch 11, **dadurch gekennzeichnet, dass** Wirkstoffe ausgewählt aus der Gruppe Antioxidatien, NO-Synthasehemmer, Catechine, Gallensäureester, Flavone, Flavonoide, Ubichinone. Plastochinone, Kreatine, Kreatinderivate und/oder Repellentien zugesetzt sind.

13. Zubereitung nach Anspruch 12 , **dadurch gekennzeichnet, dass** die Zubereitung Coenzym Q10 enthält.

14. Zubereitung nach Anspruch 11, **dadurch gekennzeichnet, dass** Lichtschutzfiltersubstanzen, die UV-A- und/oder UV-B-Strahlung absorbieren, zugesetzt sind.

15. Drug Delivery System enthaltend eine Zubereitung nach einem der vorstehenden Ansprüche.

16. Verwendung einer Zubereitung nach einem der vorstehenden Ansprüche zur Herstellung einer juckreizmindemden Creme, Lotion, Gel, Spray und/oder Schaum.

17. Verwendung einer Zubereitung nach einem der vorstehenden Ansprüche zur Herstellung eines Drug Delivery Systems umfassend die Zubereitung.

18. Verwendung einer Zubereitung nach einem der vorstehenden Ansprüche zur Herstellung eines mit der Zubereitung getränkten Tuches.

## Claims

1. Cosmetic preparation comprising
- water,
- one or more O/W emulsifiers,
- optionally one or more coemulsifiers,
- one or more oil phases
- polyethylene glycol (9) lauryl ether and
- essential oil,
**characterized in that** polyethylene glycol (12) cetearyl ether and/or monostearate polyethylene(40) is present as O/W emulsifier.

2. Cosmetic preparation based on a nonionic emulsion, comprising polyethylene glycol(12) cetearyl ether, polyethylene glycol (9) lauryl ether and menthol.

3. Cosmetic preparation according to Claim 1 comprising monostearate polyoxyethylene (40) as O/W emulsifier.

4. Preparation according to Claim 1 or 3 comprising glyceryl stearate as coemulsifier.

5. Preparation according to Claim 1, **characterized in that** the O/W emulsifiers and coemulsifiers are chosen from the group of ethoxylated fatty alcohols, ethoxylated stearates and/or glycerol esters.

6. Preparation according to one of the preceding claims comprising menthol as essential oil.

7. Preparation according to one of the preceding claims, **characterized in that** polyethylene glycol (9) lauryl ether is used in an amount of from 0.01 to 20% by weight, in particular from 1 to 10% by weight, and/or the essential oils, individually or in combination, are used in an amount of from 0.001 to 10% by weight, in particular 0.01 to 1% by weight, in the preparation.

8. Preparation according to one of the preceding claims with an oil phase content of from 1 to 50% by weight, in particular 1 to 40% by weight, based on the mass of the total preparation.

9. Preparation according to one of the preceding claims, **characterized in that** ribwort tincture is additionally present.

10. Preparation according to Claims 1 to 9, **characterized in that** the moisturizers added are polyols, such as, in particular, glycerol, sorbitol, urea and/or butylene glycol.

11. Preparation according to one of the preceding claims, **characterized in that** customary cosmetic or dermatological auxiliaries, such as dyes, pigments, powder substances, hydrophilic and/or lipophilic active ingredients, deodorants, photoprotective agents and/or other auxiliaries are added.

12. Preparation according to Claim 11, **characterized in that** active ingredients chosen from the group consisting of antioxidants, NO synthase inhibitor, catechins, gallic esters, flavones, flavonoids, ubiquinones, plastoquinones, creatines, creatine derivatives and/or repellents are added.

13. Preparation according to claim 12, **characterized in that** the preparation comprises coenzyme Q10.

14. Preparation according to claim 11, **characterized in that** photoprotective filter substances which absorb UV-A and/or UV-B radiation are added.

15. Drug delivery system comprising a preparation according to one of the preceding claims.

16. Use of a preparation according to one of the preceding claims for producing an itch-relieving cream, lotion, gel, spray and/or foam.

17. Use of a preparation according to one of the preceding claims for producing a drug delivery system comprising the preparation.

18. Use of a preparation according to one of the preceding claims for producing a cloth impregnated with the preparation.

## Revendications

1. Préparation cosmétique contenant
- de l'eau,
- un ou plusieurs émulsifiants H/E,
- éventuellement un ou plusieurs co-émulsifiants
- une ou plusieurs phases huileuses,
- de l'éther laurylique de polyéthylèneglycol (9) et
- une huile essentielle,
**caractérisées en ce que** de l'éther cétéarylique de polyéthylèneglycol (12) et/ou du monostéarate de polyoxyéthylène (40) est(sont) contenu(s) en tant qu'émulsifiant H/E.

2. Préparation cosmétique à base d'émulsion non ionique, contenant de l'éther cétéarylique de polyéthyléneglycol(12), de l'éther laurylique de polyéthylèneglycol(9) et du menthol.

3. Préparation cosmétique selon la revendication 1, comportant du monostéarate de polyoxyéthylène(40) en tant qu'émulsifiant H/E.

4. Préparation selon la revendication 1 ou 3, comportant du stéarate de glycéryle en tant que co-émulsifiant.

5. Préparation selon la revendication 1, **caractérisée en ce que** les émulsifiants H/E ou co-émulsifiants sont choisis dans le groupe des alcools gras éthoxylés, des stéarates éthoxylés et/ou des esters de glycérol.

6. Préparation selon l'une quelconque des revendications précédentes, comportant du menthol en tant qu'huile essentielle.

7. Préparation selon l'une quelconque des revendications précédentes, **caractérisées en ce qu'**on utilise dans la préparation de l'éther laurylique de polyéthylèneglycol (9) en une proportion de 0,01 à 20 % en poids, en particulier de 1 à 10 % en poids, et/ou les huiles essentielles, seules ou en association, en une proportion de 0,001 à 10 % en poids, en particulier de 0,01 à 1 % en poids.

8. Préparation selon l'une quelconque des revendications précédentes, ayant une teneur en phase huileuse de 1 à 50 % en poids, en particulier de 1 à 40 % en poids, par rapport à la masse de la préparation totale.

9. Préparation selon l'une quelconque des revendications précédentes, **caractérisée en ce qu'**on ajoute en outre une teinture de plantain lancéolé.

10. Préparation selon l'une quelconque des revendications 1 à 9, **caractérisée en ce qu'**on ajoute en tant qu'agent hydratant des polyols, comme en particulier le glycérol, le sorbitol, l'urée et/ou le butylèneglycol.

11. Préparation selon l'une quelconque des revendications précédentes, **caractérisée en ce que** sont ajoutés des adjuvants cosmétiques ou dermatologiques usuels, tels que des colorants, des pigments, des substances poudreuses, des actifs hydrophiles et/ou lipophiles, des déodorants, des photoprotecteurs et/ou d'autres adjuvants.

12. Préparation selon la revendication 11, **caractérisée en ce que** les actifs sont choisis dans le groupe des antioxydants, des inhibiteurs de NO-synthase, des catéchols, des esters d'acides biliaires, des flavones, des flavonoïdes, des ubiquinones, des plastoquinones, des créatines, des dérivés de créatines et/ou des insectifuges.

13. Préparation selon la revendication 12, **caractérisée en ce que** la préparation contient de la coenzyme Q10.

14. Préparation selon la revendication 11, **caractérisée en ce qu'**on ajoute des filtres photoprotecteurs qui absorbent le rayonnement UV-A et/ou le rayonnement UV-B.

15. Système d'apport de médicament (*Drug Delivery System*) contenant une préparation selon l'une quelconque des revendications précédentes.

16. Utilisation d'une préparation selon l'une quelconque des revendications précédentes, pour la fabrication d'une crème, d'une lotion, d'un gel, d'une composition à pulvériser en aérosol et/ou d'une mousse, atténuant les démangeaisons.

17. Utilisation d'une préparation selon l'une quelconque des revendications précédentes, pour la fabrication d'un système d'apport de médicament contenant la préparation.

18. Utilisation d'une préparation selon l'une quelconque des revendications précédentes, pour la fabrication d'une lingette imprégnée de la préparation.
